# EUROPEAN PATENT APPLICATION

(11) **EP 3 610 825 A1**
(43) Date of publication of application: **19.02.2020**
(21) Application number: 17906512.3
(22) Date of filing: 04.06.2017
(51) Int. Cl.: A61C 8/00

(54) **TOOTH OCCLUSION CONTROL HANDPIECE GUIDE PLATE AND MANUFACTURING METHOD THEREFOR**

(30) Priority: 20.04.2017 CN 201710261853
(71) Applicant: Shenzhen Aheadfit Technology Co., Ltd, Bridgehead Bao'an District Shenzhen Guangdong 518000 (CN)
(72) Inventor: WU, Shengfu, Shenzhen Guangdong 518000 (CN); WU, Meiyan, Shenzhen Guangdong 518000 (CN)
(74) Representative: Sun, Yiming
(86) International application number: PCT/CN2017/087111
(87) International publication number: WO 2018/192063

(57) **Abstract**

The present invention discloses a teeth-biting controlled handpiece head guide plate and a manufacturing method. The guide plate includes an upper matrix controlled by an upper tooth, and an upper alveolar bone or mucosa, and a lower matrix controlled by a lower tooth, and a lower alveolar bone or mucosa; a support body is disposed between the upper matrix and the lower matrix; and a control mechanism for positioning and guiding an implant handpiece head is disposed on the support body, the upper matrix or the lower matrix. When in use, the guide plate is placed between upper and lower teeth of a patient, and is fixed by enabling the upper and lower teeth to respectively bite the upper matrix and the lower matrix; and then, the handpiece head is placed into the control mechanism that is set according to implantation data, and the handpiece head is moved to an alveolar bone along a direction of the control mechanism in implant drilling. Since the guide plate guides and positions the handpiece head, the positioning and guiding accuracy is improved, the guide plate is prevented from contacting with a high-speed rotating drill bit directly, and the additional heating amount of the drill bit is not increased. The guide plate can be fixed only by biting of the patient, so that the multi-person operation in tooth implantation work turns out to be unnecessary, and the operation difficulty in tooth implantation is reduced.

## Description

### Technical Field

The present invention relates to the technical field of tooth implantation, and in particular to a teeth-biting controlled handpiece head guide plate and a manufacturing method.

### Background

A dental implant surgery may provide an important repair solution for a patient having a tooth loss. During implantation, an implant guide plate is required to help a doctor find a position, a depth and a direction of an implant in the implant surgery; and in combination with a modern digital technology, the operation time and the patient pain are reduced, and the implantation accuracy and the convenience of the implant surgery are improved.

At present, the implant guide plate consists of a matrix, one or more guide holes, a metal lantern ring in the guide hole, a hand pressing plate adapted for different drill bits, a locating bolt and other structures. The implant drilling is implemented by controlling a same guide hole on the guide plate and the hand pressing plate matched with the different drill bits according to the positioning of the guide plate, i.e., the implant drilling is implemented by controlling a position of a handpiece drill bit. However, such an implant guide plate has the following problems:
1. Fixing in-place problem: the traditional guide plate is pressed onto a tooth in place under the help of a hand of a doctor, and the hand pressing plate needs to be changed according to magnitudes of the different drill bits for hole preparation. If the guide plate is pressed unstably or rotated slightly, the guide plate is moved to cause a deviation in an implantation position. Particularly, when a mucosa supported or bone supported guide plate is used, the guide plate must be in place by using a fastening bolt. In this way, the pain of the patient is increased, and the bone burn may be generated to cause an implantation failure.
2. Accuracy problem: a core technology of the traditional guide plate is to control the drill bit via the guide hole for hole preparation. The hole preparation accuracy depends on an in-place condition of the guide plate, a height of the guide hole, and a cooperative condition among the hand pressing plate, the guide hole and the drill bit. A height of a metal sleeve is 5 mm mostly. In order not to affect the drilling of the drill bit, a diameter of the metal sleeve is 0.2 mm greater than a diameter of a matched expanding drill. As a result, a certain angle deviation of the implant is caused. Moreover, the hand pressing plate is held by the doctor and has a deviation in case of any carelessness to reduce the accuracy of the implant hole preparation.

### Technical problems

The fixation and operation are inconvenient in implant drilling, the implant drilling accuracy is not high, and the heat is not easily dissipated in drilling to cause a bone burning phenomenon.

### Summary

In order to solve the above technical problem, the present invention provides a teeth-biting controlled handpiece head guide plate, which includes an upper matrix controlled by an upper tooth, and an upper alveolar bone or mucosa, and a lower matrix controlled by a lower tooth, and a lower alveolar bone or mucosa; a support body is disposed between the upper matrix and the lower matrix; and a control mechanism for positioning and guiding an implant handpiece head is disposed on the support body, the upper matrix or the lower matrix.

Further, the control mechanism includes a fixed groove formed on the support body; a guide sleeve is disposed in the fixed groove; a fixed tube is disposed in the guide sleeve; the fixed tube and the handpiece head are fixed in the guide sleeve and are slid along a length direction of the guide sleeve; and a sleeve opening for moving a handpiece handle is formed at a side of the guide sleeve.

Further, the control mechanism includes the fixed groove formed on the support body and configured to enable the handpiece head to move smoothly along a length direction of the support body.

Further, the opening of the guide sleeve is smaller than an inner diameter of the guide sleeve.

Further, a positioning groove same as the tooth, the alveolar bone or the mucosa in shape is at least formed on the upper matrix or the lower matrix at an implant drilling side.

Further, a limit structure for limiting the fixed tube or the handpiece head to move is disposed in the fixed groove.

Further, the guide sleeve includes a metal guide sleeve.

Further, a passing hole for entering the implant handpiece head or entering the fixed tube is formed on the guide sleeve far away from an implant drilling end.

Further, a cooling groove is formed along a radial direction of an end portion of the fixed groove or/and the guide sleeve close to the implant drilling end.

The present invention further provides a manufacturing method of a teeth-biting controlled handpiece head guide plate, which includes: establishing an oral three-dimensional (3D) model: collecting oral data, and establishing the oral 3D model according to the collected oral data;
determining implant data: determining implantation data of a single implant or a plurality of implants according to the oral 3D model; and
manufacturing an implant guide plate: manufacturing an upper matrix matched with the oral 3D model and controlled by an upper tooth or an upper alveolar bone and a lower matrix controlled by a lower tooth and a lower alveolar bone, and setting a support body connecting the upper matrix and the lower matrix, where a control mechanism for positioning and guiding an implant handpiece head is disposed on the support body, the upper matrix or the lower matrix.

Further, the implantation data includes implantation position, angle and depth.

Further, the control mechanism includes a fixed groove formed on the support body; a guide sleeve is disposed in the fixed groove; a fixed tube is disposed in the guide sleeve; the fixed tube and the implant handpiece head are fixed in the guide sleeve and are slid along a length direction of the guide sleeve; and a sleeve opening for moving a handpiece handle is formed at a side of the guide sleeve.

Further, the control mechanism includes the fixed groove formed on the support body and configured to enable the implant handpiece head to move smoothly along a length direction of the support body.

Further, the opening of the guide sleeve is smaller than an inner diameter of the guide sleeve.

Further, a positioning groove same as the tooth, the alveolar bone or the mucosa in shape is at least formed on the upper matrix or the lower matrix at an implant drilling side.

Further, a passing hole for entering the implant handpiece head or entering the fixed tube is formed on the guide sleeve far away from an implant drilling end.

Further, a cooling groove is formed along a radial direction of an end portion of the fixed groove or/and the guide sleeve close to the implant drilling end.

Further, a limit structure for limiting the fixed tube or the implant handpiece head to move is disposed in the fixed groove.

### Beneficial effects

When in use, the guide plate is placed between upper and lower teeth of a patient, and is fixed by enabling the upper and lower teeth to respectively bite the upper matrix and the lower matrix, and the guide plate is provided with the control mechanism set according to implantation data; and then, the handpiece head is placed into the matched control mechanism, and the handpiece head is moved to an alveolar bone along a direction of the control mechanism in implant drilling. Since the guide plate guides and positions the handpiece head, the guide plate is prevented from contacting with a high-speed rotating drill bit directly, the deviation of drilling accuracy due to abrasion is reduced, the positioning accuracy is improved, the loss is reduced, and the additional heating amount of the drill bit is not increased. The guide plate can be fixed only by biting of the patient, so that the multi-person operation in tooth implantation work turns out to be unnecessary, and the operation difficulty in tooth implantation is reduced.

### Brief Description of the Drawings

In order to describe the technical solution in the embodiments of the present invention or in the prior art more clearly, a simple introduction on the accompanying drawings which are needed in the description of the embodiments or the prior art is given below. Apparently, the accompanying drawings in the description below are merely some of the embodiments of the present invention, based on which other drawings may be obtained by those of ordinary skill in the art without any creative effort.
FIG. 1 is a structural schematic diagram of a teeth-biting controlled handpiece head guide plate in an embodiment.
FIG. 2 is a cooperated structural schematic diagram between a teeth-biting controlled handpiece head guide plate and a tooth.
FIG. 3 is an exploded schematic diagram of a teeth-biting controlled handpiece head guide plate.
FIG. 4 is a cooperated structural schematic diagram between a teeth-biting controlled handpiece head guide plate and a handpiece.
FIG. 5 is a cooperated structural schematic diagram among a guide plate, a tooth and a handpiece.
FIG. 6 is a flowchart schematic diagram for manufacturing a teeth-biting controlled handpiece head guide plate.

The objective implementation, functional characteristics and advantages of the present invention will be further described in combination with embodiments and with references to accompanying drawings.

### Detailed Description of the Embodiments

To make the objectives, technical solution and advantages of the embodiments of the present invention clearer, a clear and complete description of the technical solution in the present invention will be given below, in combination with the accompanying drawings in the embodiments of the present invention. Apparently, the embodiments described below are a part, but not all, of the embodiments of the present invention. All of the other embodiments, obtained by those of ordinary skill in the art based on the embodiments of the present invention without any inventive efforts, fall into the protection scope of the present invention.

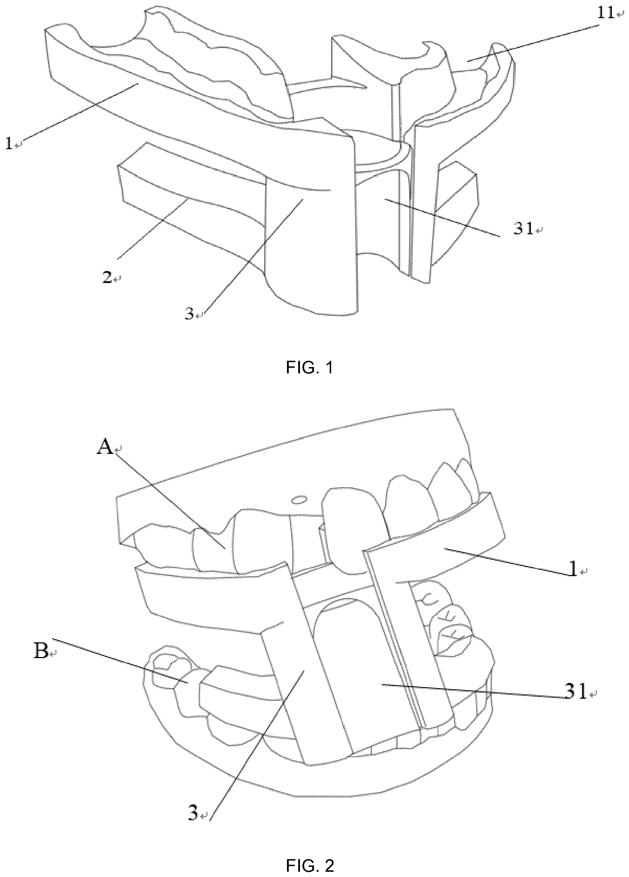

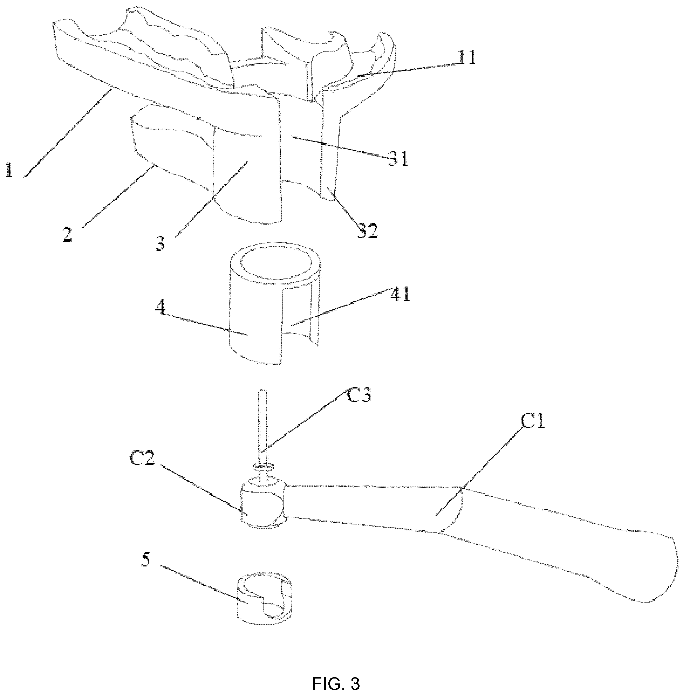

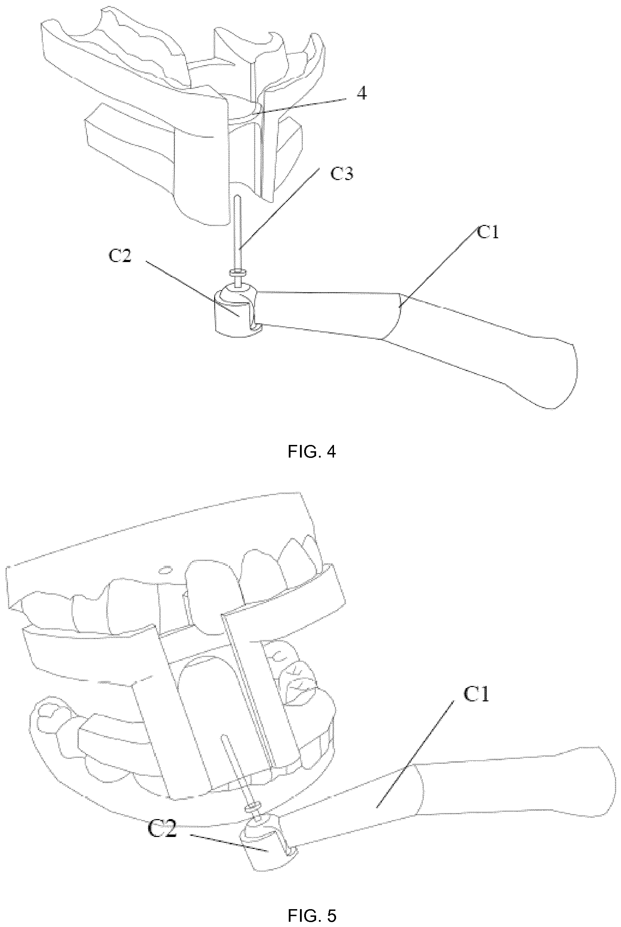

As shown in FIG. 1 to FIG. 5, the present invention provides an embodiment of a teeth-biting controlled handpiece head guide plate.

The teeth-biting controlled handpiece head guide plate includes: an upper matrix 1 controlled by an upper tooth A, and an upper alveolar bone or mucosa, and a lower matrix 2 controlled by a lower tooth B, and a lower alveolar bone or mucosa; a support body 3 is disposed between the upper matrix 1 and the lower matrix 2; and a control mechanism for positioning and guiding an implant handpiece head C2 is disposed on the support body 3.

Specifically, the implant handpiece uses the prior art and includes two types. One type is an implant handpiece with a handle, where the handle is connected to an implant handpiece head and is configured to control movement of the implant handpiece. The other type is an implant handpiece without a handle, where movement of an implant handpiece head is controlled via an external manipulator and the like. The above two handpieces are applicable. This embodiment is further described by using the first type of implant handpiece, i.e., the implant handpiece includes an implant handpiece head C2, a handpiece handle C1 and an implant drill bit C3. The implant drill bit C3 is controlled by the implant handpiece head C2.

The upper matrix 1 and the lower matrix 2 are controlled by the upper tooth A and the lower tooth B, the upper alveolar bone and the lower alveolar bone or oral upper and lower mucosas, and may also be controlled by a combination thereof.

The control mechanism includes a fixed groove 31 disposed on the support body 3. An angle of the fixed groove 31 is provided according to implantation data such as a position of an implant hole, and an angle and a depth of a dental implant. The fixed groove 31 generally consists of a circular through hole, and an open groove 32 formed on a sidewall of the circular through hole. The size of the open groove 32 is not defined, and preferably can guarantee the fixation of the guide sleeve 4, i.e., a width of the open groove 32 is smaller than a diameter of the circular through hole, so that it is assured that the guide sleeve 4 only can enter from two ends of the fixed groove 31 and is fixed with the fixed groove 31. The structure of the fixed groove 31 is not defined particularly, and the cross section may be of a circular shape and an arc shape, and may also be a regular polygonal shape, etc.

A guide sleeve 4 is disposed in the fixed groove 31, i.e., the guide sleeve 4 is fixed in the fixed groove 31; a fixed tube 5 slid along a length direction of the guide sleeve 4 is disposed in the guide sleeve 4; the fixed tube 5 is fixed with the implant handpiece head C2, so that the implant handpiece head C2 can be slid up and down synchronously along the guide sleeve 4; and when the fixed tube 5 is slid with the guide sleeve 4, the fixed tube 5 is not slid in a direction except for the length direction of the guide sleeve 4. A guide sleeve opening 41 for moving the handpiece handle C1 is formed at a side of the guide sleeve 4. The guide sleeve opening 41 is smaller than an inner diameter of the guide sleeve 4, so that the guide sleeve 4 is prevented from separating from the guide sleeve 4 when moving along the guide sleeve 4 to affect the accuracy of an implant hole. With the cooperation between the fixed tube 5 and the guide sleeve 4, shapes of different implant handpiece heads C2 may be matched to improve the applicability of the product.

The guide sleeve 4 and the fixed tube 5 are in a clearance fit, so that the fixed tube 5 may be slid along the guide sleeve 4, and a small gap between a radial direction of the fixed tube 5 and the guide sleeve 4 is prevented from affecting the implant drilling accuracy. When the fixed tube 5 is cooperatively slid with the guide sleeve 4 directly via the matched implant handpiece head C2, the implant handpiece head C2 and the guide sleeve 4 also should meet the requirement on the cooperation between the fixed tube 5 and the guide sleeve 4.

If the implant handpiece head C2 uses a structure matched with the guide sleeve 4, such as a shape rule of the implant handpiece head C2 like a cylindrical shape, the implant handpiece head C2 is not deviated when sliding along the guide sleeve 4, so that the requirement on the implant drilling accuracy may be met. In such a case, the fixed tube 5 may turn out to be unnecessary, and the implant handpiece head C2 is slid directly in cooperation with the guide sleeve 4. At this time, the guide sleeve opening 41 should be smaller than the diameter of the implant handpiece head C, so that the implant handpiece head C2 is prevented from separating from the guide sleeve 4 when moving along the guide sleeve 4 to affect the implant drilling accuracy.

When in use, the guide plate is placed between upper and lower teeth of a patient, and is fixed by enabling the upper and lower teeth to respectively bite the upper matrix and the lower matrix, and the guide plate is provided with the control mechanism set according to implantation data; and then, the handpiece head is placed into the matched control mechanism, and the handpiece head is moved to an alveolar bone along a direction of the control mechanism in implant drilling. Since the guide plate guides and positions the handpiece head, the guide plate is prevented from contacting with a high-speed rotating drill bit directly, the deviation of drilling accuracy due to abrasion is reduced, the positioning accuracy is improved, the loss is reduced, and the additional heating amount of the drill bit is not increased. The guide plate can be fixed only by biting of the patient, so that the multi-person operation in tooth implantation work turns out to be unnecessary, and the operation difficulty in tooth implantation is reduced.

As required, the control mechanism may be disposed on the upper matrix, the lower matrix or the support body provided that it can position and guide the implant handpiece head. The above embodiment is described only when the control mechanism is disposed on the support body and does not constitute into a limit for that the control mechanism is disposed on the upper matrix or the lower matrix.

In this embodiment, in order to guarantee the accuracy of the dental implant, it is necessary for the guide plate to have good stability and reliability in fixation, and may be appropriate to provide a positioning groove 11 same as the tooth, the alveolar bone or the mucosa in shape on the upper matrix 1 and the lower matrix 2. The positioning groove 11 is completely consistent with the corresponding tooth, alveolar bone or mucosa. In this way, the displacement may be prevented in biting to guarantee the accuracy of an implant drilling position. As required, it may also be appropriate to only provide the positioning groove 11 same as the tooth, the alveolar bone or the mucosa in shape on the upper matrix 1 or the lower matrix 2 at an implant drilling side, and the stability and accuracy in fixation are better than those when the positioning grooves 11 are formed on the upper and lower matrixes.

The cross section of the fixed groove 31 is not particularly defined, may be of a circular shape and may also be of a polygonal shape. No matter what shape is, the cross section of the fixed groove 31 should be matched with the guide sleeve 4, and should guarantee that the guide sleeve 4 can be fixed without moving. The material of the guide sleeve 4 is not particularly defined, and may use a metal material such as a stainless steel as required. The material of the fixed tube 5 is not particularly defined, and may also use a metal material such as a stainless steel as required.

In order to guarantee the accurate control of a depth of an implantation hole, a limit structure (not shown in the figure) for limiting the fixed tube or the implant handpiece head C2 to move is disposed in the fixed groove 41.

In order to better reduce the heat of the handpiece drill bit C3 and prevent the handpiece drill bit from overheating to cause bone burning, a cooling groove (not shown in the figure) is formed along a radial direction of an end portion of the fixed groove 31 or/and the guide sleeve 4 close to an implant drilling end, so that a cooling fluid directly enters a cooling wound, and the temperature of the implant drill bit C3 is prevented from being overhigh to cause the bone burning to make an operation failed. Meanwhile, the in-place condition of the guide plate and the condition of a surgical wound are observed conveniently in the drilling process.

Due to the cooperation among a plurality of mechanical structures, the accuracy is difficultly achieved. It may be appropriate to provide a guide structure, such as a guide strip, between the guide sleeve 4 and the implant handpiece head C2 or the fixed tube 5 to make a contact surface therebetween small and the high-precision machining more easily, and therefore the implant accuracy may be improved.

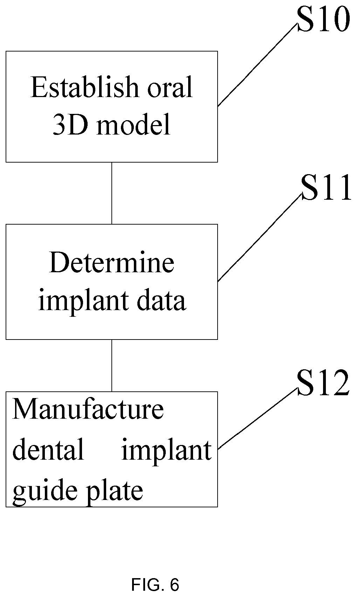

As shown in FIG. 6, the present invention further provides a manufacturing method of the teeth-biting controlled handpiece head guide plate in the above embodiment, which includes the following steps:
Step S10: establish an oral 3D model: collect oral data, and establish the oral 3D model according to the collected oral data.

Step S11: determine implant data: determine implantation data of a single implant or a plurality of implants according to the oral 3D model.

Step S12: manufacture an implant guide plate: manufacture an upper matrix matched with the oral 3D model and controlled by an upper tooth or an upper alveolar bone and a lower matrix controlled by a lower tooth and a lower alveolar bone, and set a support body connecting the upper matrix and the lower matrix, where a control mechanism for positioning and guiding an implant handpiece head is disposed on the support body, the upper matrix or the lower matrix.

Specifically, the oral data of a dental implant patient are obtained first. The oral data, such as data of a tooth and an alveolar bone, are obtained by using the prior art. The oral 3D model is established in the existing digital technology, a single implant or a plurality of implants are designed, and the implantation data are output.

Thereafter, the data of the oral 3D model and the implantation data are imported to a cutting device or a 3D printer to manufacture the dental implant guide plate. The guide plate includes an upper matrix controlled by an upper tooth and an upper alveolar bone, a lower matrix controlled by a lower tooth and a lower alveolar bone, and a support body connecting to the upper matrix and the lower matrix; a control mechanism corresponding to data of the single implant or the plurality of implants is disposed on the support body; and the control mechanism positions and guides an implant handpiece head.

The specific structure, use process and achieved technical effects of the tooth-biting controlled handpiece head are the same or similar to the above embodiment and will not be repeated herein.

The above embodiments are merely for describing the technical solution of the present invention and is not intended to limit the present invention. Although the present invention is illustrated in detail with reference to the aforementioned embodiments, it should be understood by those of ordinary skill in the art that modifications may still be made on the technical solution disclosed in the aforementioned respective embodiments, or equivalent substitutions may be made to part of the technical characteristics thereof; and these modifications or substitutions do not make the nature of the corresponding technical solution depart from the spirit and scope of the technical solution of the respective embodiments of the present invention.

## Claims

1. A teeth-biting controlled handpiece head guide plate, comprising an upper matrix controlled by an upper tooth, and an upper alveolar bone or mucosa, and a lower matrix controlled by a lower tooth, and a lower alveolar bone or mucosa, wherein a support body is disposed between the upper matrix and the lower matrix; and a control mechanism for positioning and guiding an implant handpiece head is disposed on the support body, the upper matrix or the lower matrix.

2. The teeth-biting controlled handpiece head guide plate according to claim 1, wherein the control mechanism comprises a fixed groove formed on the support body; a guide sleeve is disposed in the fixed groove; a fixed tube is disposed in the guide sleeve; the fixed tube and the implant handpiece head are fixed in the guide sleeve and are slid along a length direction of the guide sleeve; and a sleeve opening for moving a handpiece handle is formed at a side of the guide sleeve.

3. The teeth-biting controlled handpiece head guide plate according to claim 1, wherein the control mechanism comprises the fixed groove formed on the support body and configured to enable the implant handpiece head to move smoothly along a length direction of the support body.

4. The teeth-biting controlled handpiece head guide plate according to claim 1 or 2, wherein the fixed groove same as the tooth, the tooth mucosa or the alveolar bone in shape is at least formed on the upper matrix or the lower matrix at an implant drilling side.

5. The teeth-biting controlled handpiece head guide plate according to claim 1, wherein a limit structure for limiting the fixed tube or the implant handpiece head to move is disposed in the fixed groove.

6. The teeth-biting controlled handpiece head guide plate according to claim 2, wherein a passing hole for entering the implant handpiece head or entering the fixed tube is formed on the guide sleeve far away from an implant drilling end.

7. The teeth-biting controlled handpiece head guide plate according to claim 2 or 3, wherein a cooling groove is formed along a radial direction of an end portion of the fixed groove or/and the guide sleeve close to the implant drilling end.

8. A manufacturing method of a teeth-biting controlled handpiece head guide plate, comprising the steps of:
establishing an oral three-dimensional (3D) model: collecting oral data, and establishing the oral 3D model according to the collected oral data;
determining implant data: determining implantation data of a single implant or a plurality of implants according to the oral 3D model; and
manufacturing an implant guide plate: manufacturing an upper matrix matched with the oral 3D model and controlled by an upper tooth or an upper alveolar bone and a lower matrix controlled by a lower tooth and a lower alveolar bone, and setting a support body connecting the upper matrix and the lower matrix, wherein a control mechanism for positioning and guiding an implant handpiece head is disposed on the support body, the upper matrix or the lower matrix.

9. The manufacturing method of the teeth-biting controlled handpiece head guide plate according to claim 8, wherein the implantation data comprises implantation position, angle and depth.

10. The manufacturing method of the teeth-biting controlled handpiece head guide plate according to claim 9, wherein the control mechanism comprises a fixed groove formed on the support body; a guide sleeve is disposed in the fixed groove; a fixed tube is disposed in the guide sleeve; the fixed tube and the implant handpiece head are fixed in the guide sleeve and are slid along a length direction of the guide sleeve; and a sleeve opening for moving a handpiece handle is formed at a side of the guide sleeve.

11. The manufacturing method of the teeth-biting controlled handpiece head guide plate according to claim 10, wherein the control mechanism comprises the fixed groove formed on the support body and configured to enable the implant handpiece head to move smoothly along a length direction of the support body.

12. The manufacturing method of the teeth-biting controlled handpiece head guide plate according to claim 9, wherein a positioning groove same as the tooth, the alveolar bone or the mucosa in shape is at least formed on the upper matrix or the lower matrix at an implant drilling side.

13. The manufacturing method of the teeth-biting controlled handpiece head guide plate according to claim 10, wherein a limit structure for limiting the fixed tube or the implant handpiece head to move is disposed in the fixed groove.

14. The manufacturing method of the teeth-biting controlled handpiece head guide plate according to claim 10, wherein a passing hole for entering the implant handpiece head or entering the fixed tube is formed on the guide sleeve far away from an implant drilling end.

15. The manufacturing method of the teeth-biting controlled handpiece head guide plate according to claim 10, wherein a cooling groove is formed along a radial direction of an end portion of the fixed groove or/and the guide sleeve close to the implant drilling end.
